# EUROPEAN PATENT SPECIFICATION

(11) **EP 4 236 802 B1**
(45) Date of publication and mention of the grant of the patent: **17.09.2025**
(21) Application number: 21798714.8
(22) Date of filing: 26.10.2021
(51) Int. Cl.: A61B 5/282, A61B 5/257, A61B 5/00

(54) **SENSOR ARRAY AND SENSOR ARRAY SYSTEM FOR SENSING ELECTRICAL ACTIVITY**
SENSORMATRIX UND SENSORMATRIXSYSTEM ZUR MESSUNG ELEKTRISCHER AKTIVITÄT
RÉSEAU DE CAPTEURS ET SYSTÈME DE RÉSEAU DE CAPTEURS DE DÉTECTION D'ACTIVITÉ ÉLECTRIQUE

(30) Priority: 28.10.2020 EP 20382940
(43) Date of publication of application: 06.09.2023
(73) Proprietor: Corify Care S.L., 28009 Madrid (ES)
(72) Inventor: CLIMENT, Andreu, 28001 Madrid (ES); MILAGRO, Javier, 28001 Madrid (ES); HERNÁNDEZ, Erik, 48160 Derio (ES); KELLER, Thierry, 48160 Derio (ES)
(74) Representative: Balder IP Law, S.L.
(86) International application number: PCT/EP2021/079716
(87) International publication number: WO 2022/090253

(56) References cited:
- WO-A1-2010/054352
- WO-A1-2011/156105
- US-A1- 2012 200 302
- US-A1- 2016 331 263
- US-B1- 7 206 630

## Description

### TECHNICAL FIELD

The present invention relates to a device and a system for measuring electrical potentials of a body part of a patient.

### STATE OF THE ART

The acquisition of biopotentials (electric potentials of biological origin) from the surface of internal anatomical structures, such as the heart, typically involves the use of invasive measurement techniques. For example, electrophysiological studies of the cardiac tissue often rely on invasive catheters, at the distal end of which electrodes are disposed. Such electrodes are placed in contact with the tissue to record bioelectrical activity at that location (for example, the heart).

Non-invasive measurement techniques can also be employed. These are usually based on sensors placed on the body surface. If the geometry and location of the internal anatomical structure is known and the position of the surface sensors identified, it is possible to use surface biopotentials to calculate, using a variety of mathematical methods, the electrical activity in the internal structure. The use of non-invasive measurement techniques has large benefits in terms of patient's comfort and safety and economic and time costs.

An example of non-invasive measurement system is disclosed in US2016/0331263A1, which discloses a customizable electrophysiological mapping electrode patch system composed of several sensor patches made of a flexible material to be mounted on the torso of a patient. Each patch is made of an upper planar layer and a lower planar electrically insulative layer. Sensing electrodes are disposed on the upper layer or between the two layers. Specific pre-cuts are defined in the patch. However, these pre-cuts do not enable to change the relative position of the electrodes remaining in the patch after the pre-cut portions are removed.

In turn, US2013/0281814-A1 discloses a sensor apparatus dimensioned and configured to be disposed on the torso of a patient, thus forming a vest. It is made of a flexible substrate layer on which an electrode layer is disposed. Electrode layer includes a distributed arrangement of electrodes and corresponding electrically conductive traces, forming loops. The flexible substrate layer is mounted to a stretchable-fabric layer which enables the vest to highly conform to the patient's body shape and movements. In order to accommodate a range of patient's sizes and body types, a plurality of sizes are provided.

In WO2010/054352A1, a sensor array system includes elongated strips of flexible material. Electrically conductive sensors are distributed along the length of each elongated strip, providing columns of sensors. Elongated strips are connected together by connecting elements which can be cut or torn in order to reposition columns of sensors relative to each other. However, due to the configuration of sensor strips forming columns, sensor repositioning is limited to complete strips (that is to say, columns of sensors).

Document WO 2011/156105 A1 discloses a customizable medical electrode array comprising a plurality of cut-outs establishing a pattern of islands interconnected by conductive member bridges.

State-of-the-art sensor array systems have limited sensor repositioning capabilities, both in terms of modifying the position of the complete set of electrodes on the patient's body part, and in terms of reducing the total amount of electrodes in the array.

Therefore, there is a need to develop an improved sensor apparatus that overcomes the drawbacks of conventional ones.

### DESCRIPTION OF THE INVENTION

The present invention is defined by the appended claims 1-15.

The device and system for measuring electrical potentials of a body part of a patient described in the present invention intends to solve the shortcomings of prior-art devices and systems therefor. They enable the acquisition of surface bioelectrical potentials of a patient while enable large sensor repositioning capabilities. This allows, for example, providing a unique device (i.e. sensor array) for a wide spectrum of patient's sizes and complexions; or the simultaneous attachment of additional sensors or sensing patches intended for other purposes, such as those used in a variety of medical equipment.

A first aspect of the invention relates to a device for measuring electrical potentials of a body part of a patient. The device comprises: a plurality of substrate nodes made of a flexible material, the plurality of substrate nodes being configured to be disposed on the torso of the patient, wherein some pairs of substrate nodes of the plurality of substrate nodes are interconnected by straight portions of flexible material, the plurality of substrate nodes and the straight portions of flexible material forming a flexible substrate, wherein there is one straight portion per pair of substrate nodes to be interconnected; a plurality of electrodes, wherein each electrode of the plurality of electrodes is disposed on a respective substrate node of the plurality of substrate nodes; and at least one connector. Each electrode of the plurality of electrodes is connected to the at least one connector through a respective conductive path, wherein each conductive path is embedded in the flexible substrate formed by substrate nodes and straight portions. Some of the straight portions connecting two substrate nodes have an embedded conductive path. The device is configured to remove one or more substrate nodes and corresponding electrodes by cutting the portions which connect the one or more substrate nodes and corresponding electrodes with the remaining substrate nodes in the device, without interrupting the conductive paths of the remaining electrodes in the device.

In embodiments of the invention, the substrate nodes have circular shape.

In embodiments of the invention, each substrate node is connected to two or three substrate nodes by respective two or three straight portions.

In embodiments of the invention, each substrate node is connected to between two and six adjacent nodes by a respective number of straight portions.

In embodiments of the invention, groups of substrate nodes and respective electrodes are aligned forming columns.

In embodiments of the invention, neighbor substrate nodes and respective electrodes belonging to different columns are disposed in zig-zag.

In embodiments of the invention, for all inner substrate nodes, each substrate node is surrounded by six neighboring substrate nodes, each neighboring substrate node being disposed in a corner of an imaginary hexagon centered around the substrate node.

In embodiments of the invention, the device is configured for placement on the anterior portion of a user's torso, in which case the device comprises five columns of substrate nodes and corresponding electrodes, wherein a first column has five substrate nodes, a second column has five substrate nodes, a third column has nine substrate nodes, a fourth column has eight substrate nodes and a fifth column has eight substrate nodes.

In embodiments of the invention, the device is configured for placement on the posterior portion of a user's torso, in which case the device comprises four columns of substrate nodes and corresponding electrodes, wherein a first column has five substrate nodes, a second column has eight substrate nodes, a third column has eight substrate nodes and a fourth column has eight substrate nodes.

In embodiments of the invention, the width of each portion connecting two substrate nodes depends on the number of conductive paths it carries.

In embodiments of the invention, the at least one connector is connected to an acquisition system for further processing of the sensed electrical potentials.

In embodiments of the invention, the device further comprises a substrate portion disposed between the at least one connector and one of the substrate nodes, said substrate portion carrying a portion of all the conductive paths connecting the electrodes with the at least one connector.

In embodiments of the invention, the device further comprises a unique identifier associated with at least some of the electrodes, the unique identifier enabling automatic identification of the location of electrode with which it is associated.

In embodiments of the invention, the unique identifier is a visual code.

In embodiments of the invention, the device further comprises at least one additional substrate node and corresponding electrode serving as electrical reference.

A second aspect of the invention relates to a system comprising at least two devices of the first aspect of the invention.

A third aspect (not claimed) relates to the use of at least one device of the first aspect of the invention, for measuring electrical potentials of a body part of a patient.

Unlike conventional sensor systems in which the electrodes are disposed in strips forming columns and therefore repositioning of electrodes within a column is extremely scarce, in the present invention, the position of every single electrode can be modified with respect to the position of neighbouring electrodes. This is enabled by a combination of features: the nodes - having respective electrodes- are isolated items connected to other nodes through a portion of flexible material; nodes -and corresponding electrodes- can be removed from the device without affecting the operation of the remaining electrodes; and the absence of electrical routes in certain portions connecting nodes, which permits that, when these portions are cut in order to increase the repositioning capacity of the affected nodes, remaining nodes are not disabled. The device is one size, which facilitates its manufacturing, but can be adapted to patients of different sizes and shapes thanks to high reconfiguration capabilities derived from the isolated nodes carrying the electrodes, the thin flexible portions connecting nodes and the lack of electrical routes in certain connecting portions.

Besides, because neighbor electrodes belonging to different columns are not aligned, but disposed in zig-zag, a larger surface can be covered. In addition, the effective area of the array support or substrate is minimized, because the substrate is merely formed by single nodes plus elongated portions interconnecting nodes. This contributes also to increase the repositioning capabilities of the device, facilitating the rearrangement of electrodes on the patient's body part with more freedom. This mesh topology maximizes the void space (or surface) between nodes (and therefore electrodes), leaving therefore a large surface for accessing the patient's body part (for example torso) if needed.

Additional advantages and features of the invention will become apparent from the detailed description that follows and will be particularly pointed out in the appended claims.

### BRIEF DESCRIPTION OF THE DRAWINGS

The drawings comprise the following figures:
FIG. 1a shows a sensor array designed to cover the left anterior portion of a patient's torso, according to a possible embodiment of the invention.
FIG. 1b shows a sensor array designed to cover the right anterior portion of a patient's torso, according to a possible embodiment of the invention.
FIG. 1c shows a sensor array designed to cover the right posterior portion of a patient's torso, according to a possible embodiment of the invention.
FIG. 1d shows a sensor array designed to cover the left posterior portion of a patient's torso, according to a possible embodiment of the invention.
FIG. 1e shows a sensor array designed to cover the left anterior portion of a patient's torso, according to a possible embodiment of the invention.
FIG. 1f shows a sensor array designed to cover the right anterior portion of a patient's torso, according to a possible embodiment of the invention.
FIG. 2a shows a lateral view of different layers forming a sensor array according to a possible embodiment of the invention.
FIG. 2b shows a top view of different layers forming a sensor array according to a possible embodiment of the invention.
FIG. 3 shows a view of a sensor array according to the invention, disposed on the torso of a patient.
FIG. 4 shows a detailed view of a piece of a sensor array according to embodiments of the invention.
FIG. 5a shows a detailed view of a portion of a sensor array according to embodiments of the invention, in which two possible cutting zones are identified. By cutting the sensor array by a cutting zone, certain sensors can be removed from the array without affecting other sensors in the array.
FIG. 5b shows a detailed portion of the sensor array of FIG. 1a, in which some nodes and corresponding electrodes, portions and conductive paths are shown in detail.
FIG. 6 shows the sensor array of FIG. 1a, but all the electrodes have been identified in order to explain the design criteria of the electrical conducting paths according to a possible embodiment of the invention.
FIG. 7a shows the bottom side of a sensor array designed to cover the left anterior portion of a patient's torso, according to a possible embodiment of the invention.
FIG. 7b shows the top side of a sensor array designed to cover the left anterior portion of a patient's torso, including a possible set of codes used to unambiguously identify the position of each node, and therefore electrode, according to an embodiment of the invention.

### DESCRIPTION OF A WAY OF CARRYING OUT THE INVENTION

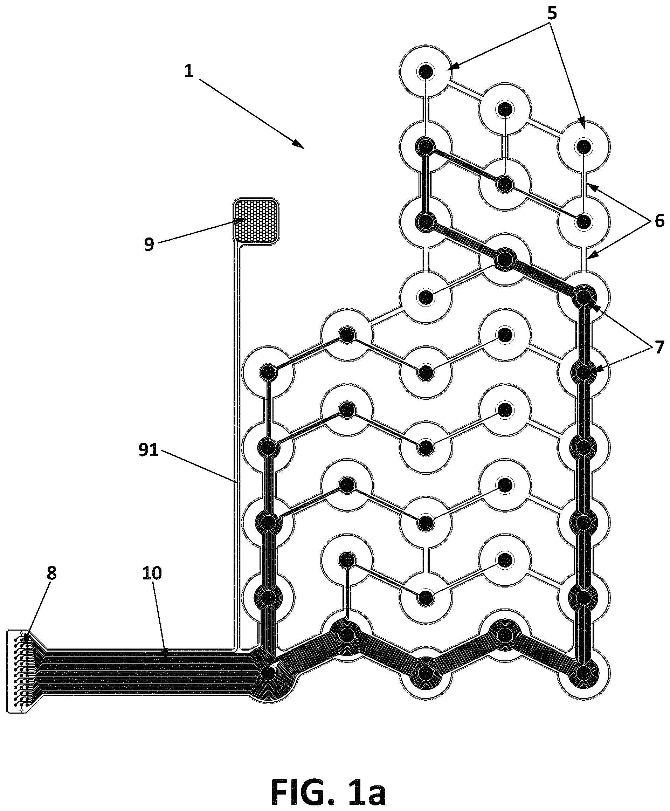

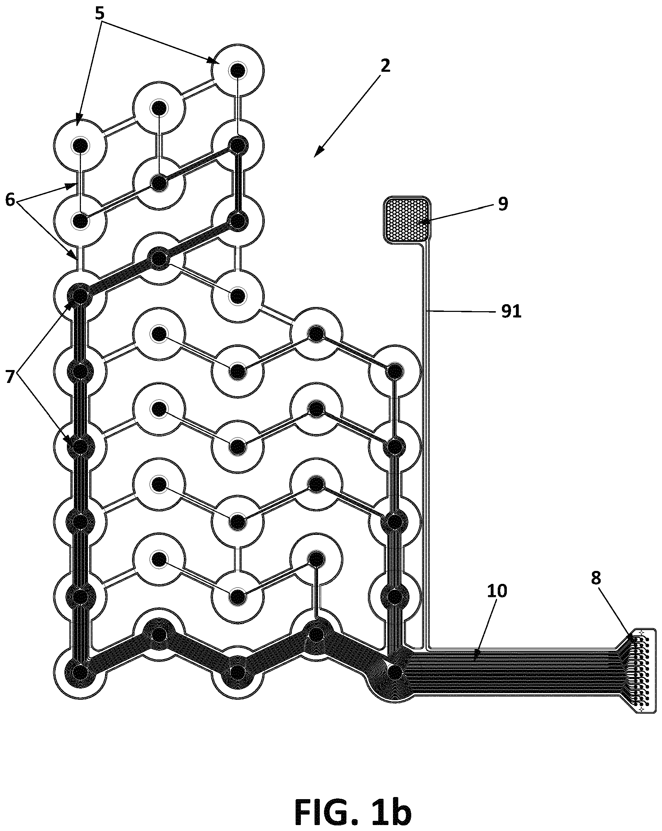

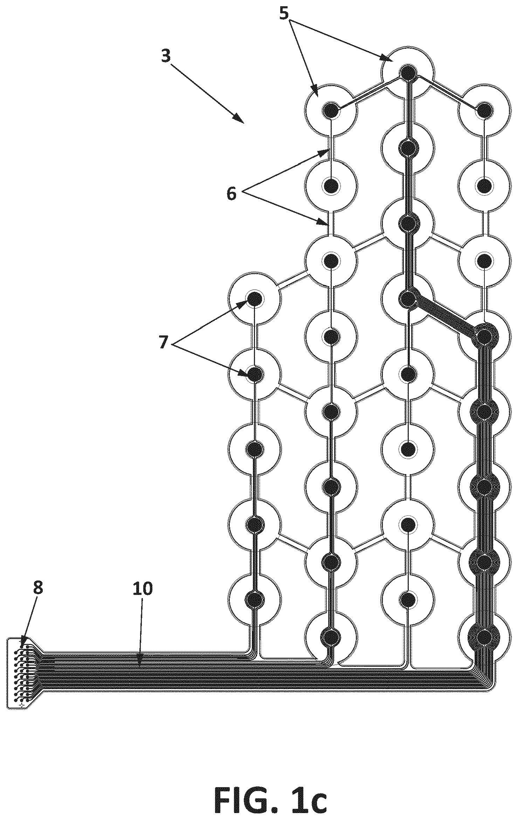

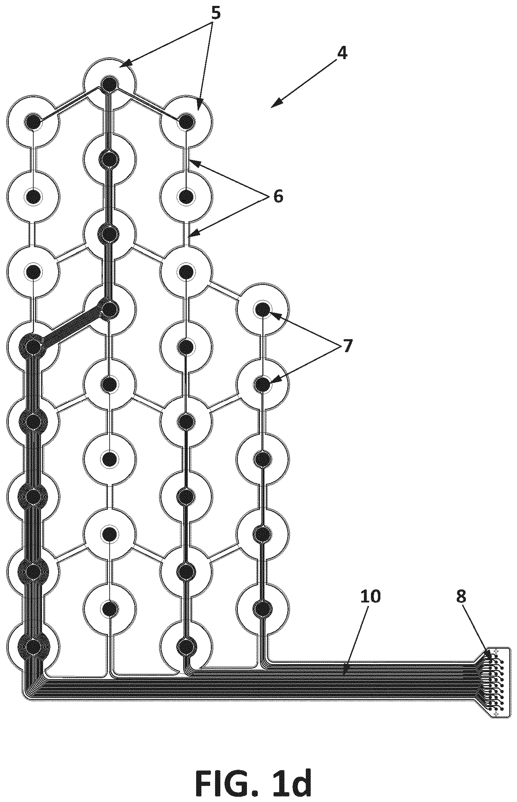

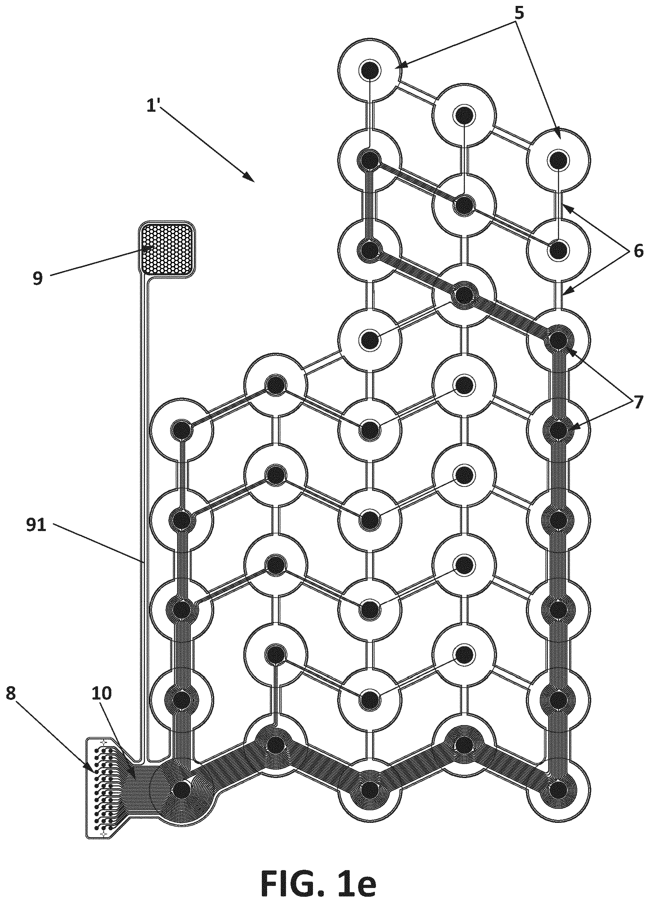

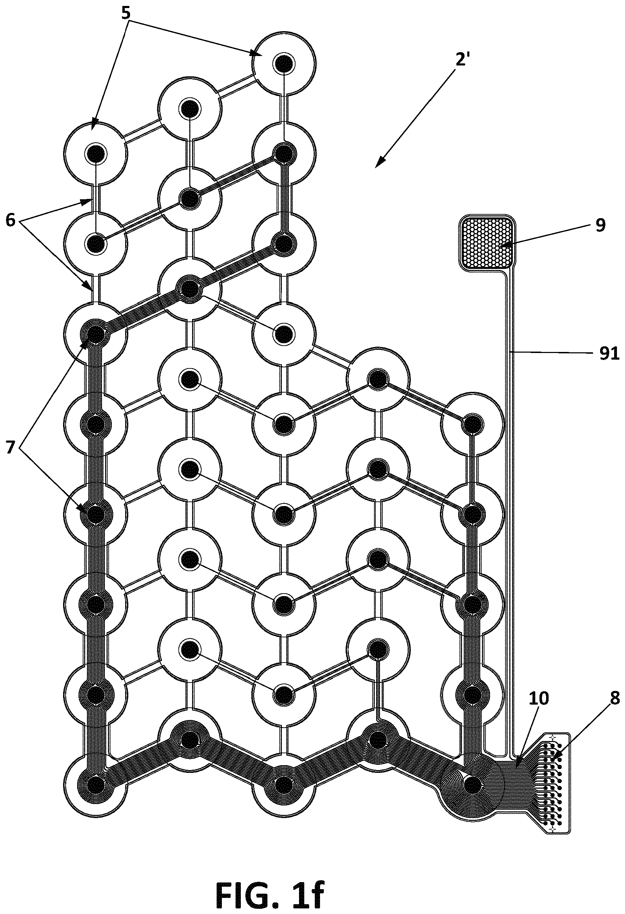

FIGs. 1a-f schematically show respective embodiments of six devices according to the invention. The devices are electrode arrays, also referred to as sensor arrays, designed to cover an anatomical structure of the patient, such as the torso. Each device 1-4, 1', 2' is dimensioned and configured for placement on a different anatomical region of a patient's body. The shown electrode arrays are designed to be positioned on the left anterior portion of a patient's torso (device 1, FIG. 1a or device 1', FIG. 1e), the right anterior portion thereof (device 2, FIG. 1b or device 2', FIG. 1f), the right posterior portion thereof (device 3, FIG. 1c) and the left posterior portion thereof (device 4, FIG. 1d). At least two of the four devices (sensor arrays) may form a sensor array system. In other words, a sensor array system may be formed by several devices (i.e. sensor arrays), thus covering a larger portion of a patient's body. The devices 1-4, 1', 2' are in the form of flexible and pliant substantially planar elements adaptable to the curvature of a patient's anatomy to which they are placed.

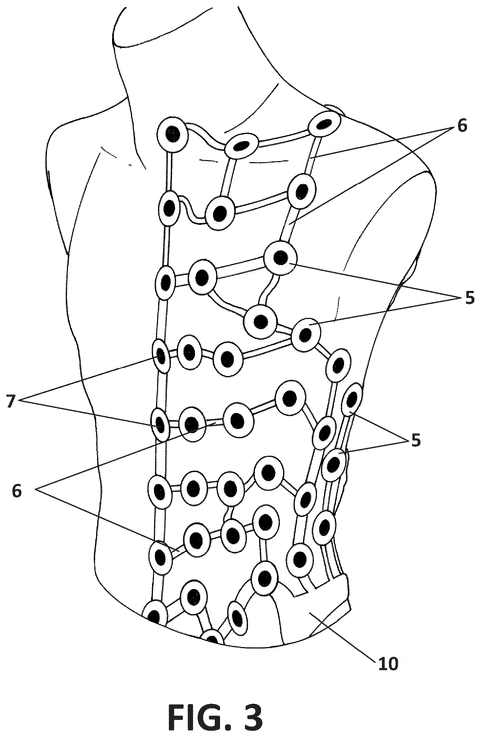

Electrode arrays 1-4, 1', 2' form, together with other electro-mechanical components, such as one or more power supply, cables, connectors, controlling means, other transducers and/or ground electrodes, a mapping electrode system for measuring surface biopotentials of a patient, which can be employed for assessing the electrical activity of an internal anatomical structure in a non-invasive way. For example, the shown electrode arrays 1-4, 1', 2' may be designed for non-invasive electrophysiological mapping of a patient's heart activity. A surface electrocardiographic map may be obtained from data (i.e. electrical signals) captured by a number of surface electrodes of electrode arrays 1-4, 1', 2' , by associating the captured data with a particular area of the patient's body. In FIG. 3, a side view of a sensor array disposed on, adhered or otherwise affixed to the torso of a patient is shown.

Each electrode array 1-4, 1', 2' comprises a plurality of electrodes 7. Electrodes 7 are electrical sensing electrodes, also referred to as electrically conductive sensors. Electrodes 7 are configured to sense electrical activity originating in the body of a patient. Electrodes 7 are arranged in a generally even distributed relationship according to the anatomical region for which they have been designed and over which they are going to be positioned. Different sensor arrays 1-4, 1', 2' may have different numbers and distributions of electrodes 7 depending for example on where the respective sensor arrays are to be positioned (the right or left anterior portion of the torso, the right or left posterior portion thereof, etc.).

Electrodes 7 are disposed on a substrate made of flexible material. The substrate is composed of individual nodes (substrate nodes) 5 interconnected by portions (substrate portions or substrate interconnections) 6 of flexible material. Substrate nodes 5 and substrate portions 6 are preferably made of a same flexible material. Nodes 5 provide an electrode-carrying substrate layer. The substrate forms a mesh of substrate nodes 5 interconnected by portions 6. Each electrode 7 of an electrode array 1-4, 1', 2' is disposed on a corresponding node 5. There is one electrode per substrate node. There are as many electrodes as substrate nodes. Nodes 5 are islands of flexible material, meaning that nodes 5 do not directly touch any other node 5. This provides great flexibility to the sensor array 1-4, 1', 2' .

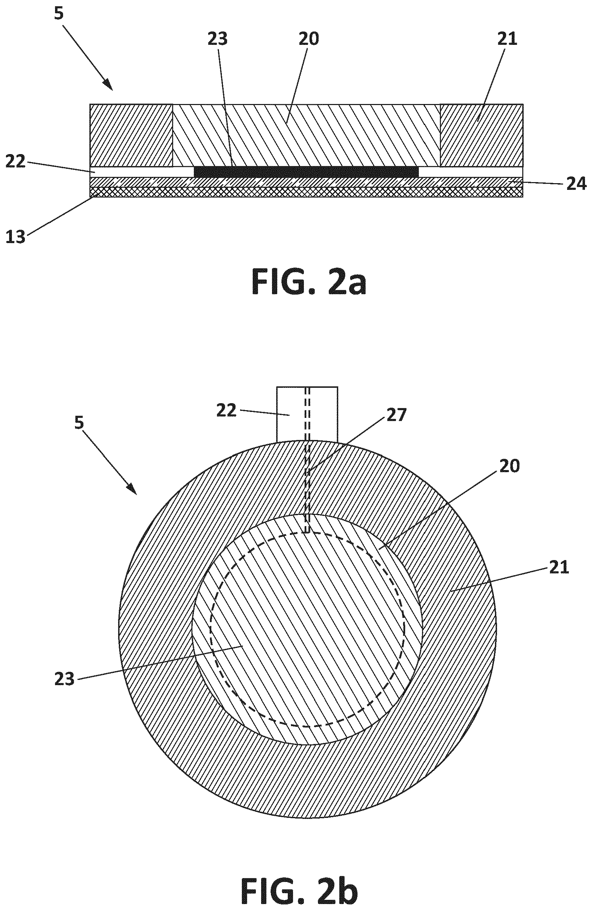

The lateral and top views of FIGs. 2a-b show different layers of which each node 5 and corresponding electrode 7 may be composed of in a possible embodiment of the invention. The structure of a node 5 has a flexible substrate layer 24. Non-limiting examples of flexible materials of which the substrate layer 24 may be made of are: plastic or polymers, such as polyester, for example polyethylene terephthalate (PET), thermoplastic polyurethane (TPU), or other flexible materials, such as cellulose, waxed paper or elastane. In embodiments of the invention, the thickness of the flexible substrate layer 24 may vary between 100 and 150 µm (micrometers, 10⁻⁶ meters). Substrate interconnections 6 (FIGs. 1a-f) are preferably made of the same flexible material of which substrate layer 24 is made. An electrode layer 23 is disposed or deposited onto the substrate layer 24. The electrode layer 23 can be made of different conductive materials, such as metals, metal alloys, carbon-based materials and combinations thereof. Non-limiting examples of metals that may be used are gold, silver and copper. In a possible embodiment, the electrode layer 23 (which corresponds to electrode 7 in FIGs. 1(a-f)) may be made of a two-layer conductor combining a metal and a metal alloy, for example Ag/AgCl. The electrode layer 23 can be surrounded by a dielectric layer 22, which can be made of a variety of dielectric materials. Electrical paths or tracks 27 will preferably be deposited/printed on the substrate layer.

The node 5 and electrode 7 may comprise other optional layers, such as an electrode-skin interface layer and/or an adhesive layer. An electrode-skin interface layer 20 may be applied on top of the electrode layer 23 and the dielectric layer 22. The electrode-skin interface layer 20 may be a conventional hydrogel layer. The presence or absence of electrode-skin interface layer 20 may depend, for example, on the type of electrodes used. On top of the electrode layer 23 and the dielectric layer 22, surrounding the electrode-skin interface layer 20 (if there is layer 20), there may be an adhesive layer 21 for attaching the node 5 and electrode 7 to the patient's skin. The adhesive layer contributes to fixing the node 5 and electrode 7 to the patient's skin. In FIGs. 2a-b, there is an electrode-skin interface layer 20 surrounded by an adhesive layer 21. The adhesive layer 21 can be made of adhesive insulating foam. When an adhesive layer 21 is used, the adhesive layer 21 is preferably not in contact with the electrode layer 23. Therefore, the interface between the electrode layer 23 (conductor) and the patient's skin is either the air or a hydrogel layer. In embodiments in which there is no adhesive layer 21, the electrodes 5 may attach to the patient's skin by means of the electrode-skin interface layer 20, if there is one, since certain hydrogel layers may act also as fixing means due to their adhesive properties. As shown in FIG. 2a, a visual code 13 may be printed on or attached to the opposite surface of the substrate layer 24 on which the electrode layer 23 is placed. In other words, a visual code 13 may be disposed on the outer surface of the substrate layer 24 in the node 5.

Nodes 5 are connected by flexible portions (flexible interconnections) 6. Flexible portions 6 maintain the mesh structure and the distribution and orientation of nodes 5. Flexible portions 6 are substantially flat. Each flexible portion 6 is used to interconnect a pair of nodes 5. Portions 6 are straight, that is to say, they do not define curved trajectories between pairs of nodes 5. Not all pairs of neighboring nodes 5 are interconnected by portions 6. Flexible portions 6 enable the repositioning of the electrodes 7 (or at least of some of them) when required. For example, a patient may need a larger density of electrodes disposed on his/her torso, with respect to another patient (for example depending on the type of measurement to be taken, or on the size of the patient). When a larger density of electrodes is required, nodes 5 (with their respective electrodes 7) may be disposed closer by on the patient's torso, in which case flexible portions 6 may be bended on (above) the patient's torso. Instead, when a lower density of electrodes is required, nodes 5 (with their respective electrodes 7) may be disposed more separate from each other on the patient's torso, in which case flexible portions 6 may be smoothed (without folds or curves) on the patient's torso.

Substrate nodes 5 are substantially flat and may have any shape, such as circular (disc-shaped), oval, square, rectangular or any other shape. In the shown embodiments they are circular. The diameter of the substrate nodes 5 (or any other equivalent dimension if the nodes 5 are not circular) may vary between 30 and 60 mm (millimeters, 10⁻³ meters), such as between 35 and 55 mm, or between 40 and 50 mm. The diameter of the electrode contact region (diameter of electrode 7) may vary between 5 and 20 mm, such as between 7 and 17 mm, or between 10 and 15 mm.

Each substrate node 5 is connected to at least two other adjacent nodes 5 by respective at least two portions or interconnections 6. In some embodiments, each node 5 is connected to a maximum number of three adjacent nodes by a respective number of portions 6. In some embodiments, each node 5 is connected to between two and six adjacent nodes by a respective number of portions 6. For example, a majority of nodes 5 (for example more than 75% of the nodes, or more than 80% of the nodes, or more than 85% of the nodes, or more than 90% of the nodes) is connected to a maximum number of four adjacent nodes, while some other nodes 5 are connected to five or even to six adjacent nodes. Connections or portions 6 between adjacent nodes provide the system with a stable structure, whereas the total number of connections 6 is limited to maximize the void space between nodes 5 so that the anatomical structure to which the system is attached can be easily accessed by medical personnel. Additionally, the absence of connection between some pairs of adjacent nodes enables to vary the relative positions of such nodes easily. Positioning and repositioning of electrodes 7 is therefore enabled by a combination of features: (1) each node 5 holds a single electrode 7, and therefore by moving a single node 5, only its corresponding electrode 7 is repositioned; (2) flexible portions or interconnections 6 interconnecting substrate nodes 5 enable repositioning of single nodes 5 (and therefore the respective electrodes 7 attached to the nodes) while other electrodes 7 (in other nodes 5) remain attached to the body part of the patient. In particular, repositioning of electrodes is much more flexible in the proposed sensor array 1-4, 1', 2' than in conventional arrays in which electrodes are arranged on a substrate layer, for example forming a patch or a strip of electrodes, or in conventional arrays in which electrodes are connected by a long portion used to interconnect a plurality of electrodes. Besides, the fact that, in some embodiments, each substrate node 5 is only physically interconnected to a maximum of three neighboring or adjacent nodes 5 through corresponding portions 6, or in some embodiments a majority of nodes 5 is connected to a maximum number of four nodes 5, while only a few nodes 5 are physically connected to five or six neighboring nodes 5, enables the repositioning of single electrodes 7 -by repositioning the substrate node 5 to which they are attached-, without affecting the position of neighboring electrodes 7. Repositioning certain electrodes 7 may facilitate the simultaneous use of the sensor array 1-4, 1', 2' and other systems which may also require attaching something (i.e. patches) to patient's skin. Non-limiting examples of such other systems are catheter mapping systems or defibrillators. Positioning and repositioning of electrodes 7 is enabled by an additional feature: the absence of electrically conductive path in some portions 6- which is described later.

In the embodiments shown in FIGs. 1a-f the electrodes of each electrode array 1-4, 1', 2' form a substantially regular mesh of electrodes 7 (and corresponding substrate nodes 5). The regular mesh of electrodes comprises several columns of electrodes. In other words, electrodes 7 (or substrate nodes 5) are vertically aligned (aligned in columns). For example, electrode arrays 1-2 and 1'-2' (FIGs. 1a-b, e-f) form five columns, while electrode arrays 3-4 (FIGs. 1c-d) form four columns. Electrode arrays 1-2 are symmetric. Electrode arrays 3-4 are also symmetric. Electrode arrays 1'-2' are symmetric. In a column, when two neighboring substrate nodes 5 are connected by a portion 6, the two nodes 5 and interconnecting portion 6 form a straight (vertical) strip. However, not all pairs of neighbor nodes 5 in one column are interconnected by a portion 6. In the electrode arrays 3-4, 1', 2' embodied in FIGs. 1c-f, in each column each pair of neighboring nodes 5 forming the column is connected by a portion 6. This improves the adjustment of the array to the user's torso. In turn, substrate nodes 5 (and corresponding electrodes 7) belonging to neighbor columns are not aligned but are rather disposed following a zig-zag pattern. The zig-zag pattern enables: (1) to maximize the area (surface of patient's body part) covered by the electrode array; (2) to maximize the void area on the surface of the patient's body part; and (3) to reposition nodes 5 in an optimal way.

In the embodiments shown in FIGs. 1a-f, except for the outer nodes 5 of each electrode array 1-4, 1', 2', each node 5 (inner nodes) is surrounded by six neighboring nodes, each neighboring node being disposed in a corner of an imaginary hexagon centered around the mentioned node. Each node 5 is only interconnected (by a corresponding number of portions 6) to two or three of the six neighboring nodes. This contributes greatly to the repositioning capacity of nodes 5. Due to their configuration as perimeter nodes, outer nodes 5 are only surrounded by two to four neighboring nodes. Outer nodes are also interconnected (by a corresponding portion 6) to two or three neighboring nodes.

Because neighbor electrodes belonging to different columns are not aligned, but disposed in zig-zag, a larger surface can be covered. In addition, the effective area of the array support or substrate (nodes 5 and portions 6) is minimized, because the substrate is merely formed by single nodes plus elongated portions interconnecting nodes. This contributes also to increase the repositioning capabilities of the device, facilitating the rearrangement of electrodes on the patient's body part with more freedom. This mesh topology maximizes the void space (or surface) between nodes (and therefore electrodes), leaving therefore a large surface for accessing the patient's body part (for example torso) if needed.

In the electrode arrays 1 and 1' of FIGs. 1a and 1e, the first column has five electrodes, the second column has five electrodes, the third column has nine electrodes, the fourth column has eight electrodes and the fifth column has eight electrodes. Therefore, in the respective symmetric electrode arrays 2 and 2' of FIGs. 1b and 1f, the first column has eight electrodes, the second column has eight electrodes, the third column has nine electrodes, the fourth column has five electrodes and the fifth column has five electrodes.

In the electrode array 3 of FIG. 1c, the first column has five electrodes, the second column has eight electrodes, the third column has eight electrodes and the fourth column has eight electrodes. Therefore, in the symmetric electrode array 4 of FIG. 1d, the first column has eight electrodes, the second column has eight electrodes, the third column has eight electrodes and the fourth column has five electrodes.

Because nodes are not connected to all their neighbouring nodes, the relative position of nodes with respect to neighbouring nodes can be easily modified to, for example, increase the existing space between nodes to attach other medical equipment to the patient, or reduce it to increase electrode density in the vicinity of a determined anatomical structure.

Electrodes 7 are connected to a connector 8 through electrically conductive paths. Connector 8 may be electrically linked -typically by a connection cable- to an acquisition system (not shown) for further processing of the sensed biopotentials. Connector 8 is configured to receive stimuli from the electrodes 7 through conductive paths.

In particular embodiments, one or more nodes 9 (including corresponding electrodes) may be used to set an electrical reference. Nodes 9 are therefore referred to as reference nodes 9. In the embodiments shown in FIGs. 1a-b and 1e-f, reference node 9 is implemented as an additional patch having an electrode, directly connected to portion 10 by a portion 91 of flexible material. A conductive path disposed on the portion provides electrical connection between patch 9 and connector 8. In FIGs. 1a-b and 1e-f, reference nodes 9 are not connected to any node 5 of the mesh, so that they can be placed in a completely different location, far from the rest of electrodes 7. In the examples of FIGs. 1a-b and 1e-f, reference electrodes 9 are connected to portion 10 by a straight portion 91 of a substrate material, which can be a plastic or a polymer, such as a polyester, for example **PET,** TPU, or other flexible material, such as cellulose, waxed paper or elastane. In other possible embodiments, reference nodes 9 may be connected to portion 10 using flexible wires, cables, or other electrical conductors. Reference nodes 9 are substantially flat and may have any shape, such as circular (disc-shaped), oval, square, rectangular or any other shape. In the shown embodiments they are square-shaped.

A different electrically conductive path connects each electrode 7 with a connector 8. There are as many conductive paths as electrodes 7. Conductive paths are illustrated in FIGs. 1a-f as black lines connecting electrodes. The manufacturing of the electrodes and portions between the electrodes can be achieved, e.g., by screen-printing. In one possible manufacturing process, the electrode layer 23 and/or the electrically conductive paths can be printed on top of a previously printed substrate layer 24. Afterwards, the dielectric layer 22 can be printed on top of the electrode layer 23 and/or the electrically conductive paths. The adhesive layer 21 and/or the electrode-skin interface layer 20 can be printed on or attached to the dielectric layer 22 as appropriate. Finally, visual codes 13 can be printed on or attached to the opposite face of the substrate layer 24 to which the electrode layer 23 and/or the electrically conductive paths were printed on. Printing screens can be employed to delimit the region on which each layer must be printed.

Conductive paths may be disposed on or embedded in the flexible material forming the substrate nodes 5 and interconnecting portions 6 in different ways. In one embodiment, conductive paths may be formed by printing or inking an electrically conductive and bendable or flexible material onto nodes 5 and portions 6, 10. For example, conductive paths may be formed by screen printing with a thickness of between 0.01 mm and 0.04 mm and a width of about 0.5, with a separation between conductive paths of about 0.5 mm. Alternatively, conductive paths may be formed by integrating electrically conductive yarns into the material forming the nodes 5 and portions 6, 10. Yarns may have a section (i.e. diameter) of between 0.02 mm and 1 mm. Conductive materials forming conductive paths may also be adhered or glued to nodes 5 and portions 6, 10. Conductive paths may be made of different conductive materials, such as gold, silver, copper, carbon, metals or metal alloys. In other embodiments, conductive paths may comprise wires, cables, ribbon conductors, flexible electronic circuits, flex circuits and flexible plastic substrates with electrical conductors disposed therein or thereon.

In one embodiment, the distal end of each conductive path is attached to an electrode 7, whereas the proximal end thereof is attached to a proximal electrical connection (referred to as 8 in FIGs. 1a-f or tab configured so that an external electrical connector can be attached to it, where such connector is, for example, a connector of a mapping cable or any another interface with an acquisition system. In the shown embodiments, all electrodes 7 in a sensor array 1-4, 1', 2' have their distal end attached to a same proximal electrical connection 8. However, in other embodiments, different groups of electrodes may be attached to different electrical connections. Conductive paths travel from each electrode 7 to a proximal electrical connection 8 along some of the portions 6 and substrate nodes 5, in such a way that all the electrodes 7 are connected to a proximal electrical connection by at least one conductive path. A portion of all these conductive paths travel towards electrical connection 8 on substrate portion 10, which comprises the proximal end of all conductive paths.

In the shown examples, the substrate portion 10 is made of the same material as portions 6, but in other embodiments it could comprise flexible wires, cables, or other electrical conductors presented individually or embedded within a flexible structure such as a cover made of polyvinyl chloride (PVC), polyethylene (PE), nylon or other thermoplastic or similar flexible materials.

In general, different interconnecting portions 6 contain a different number of conductive paths. The electrode arrays 1-4, 1', 2' are designed in such a way that a majority of conductive paths are implemented in portions 6 disposed at the periphery of the electrode array. Having as few conductive paths as possible in inner portions 6 of the electrode array enables the removal of nodes and corresponding electrodes (by cutting certain portions 6) without affecting the operation of remaining electrodes. This is shown in FIGs. 1a-f, which show all the conductive paths of electrodes arrays 1-4, 1', 2' . Thus, the outer nodes and corresponding interconnection portions contribute more to a structural function of the electrode array than the inner nodes, while the inner nodes provide great versatility in terms of repositioning or removal. The width of each portion 6 connecting two substrate nodes 5, and carrying conductive paths, may vary, depending for example on the number of conductive paths they need to carry. For example, the width of the substrate portions 6 can vary from less than 8 mm, such as less than 6 mm (for example 4 mm) for portions carrying one or zero conducting paths, to more than 30 mm, such as more than 35 mm (for example 39 mm) for portions carrying 36 conducting paths.

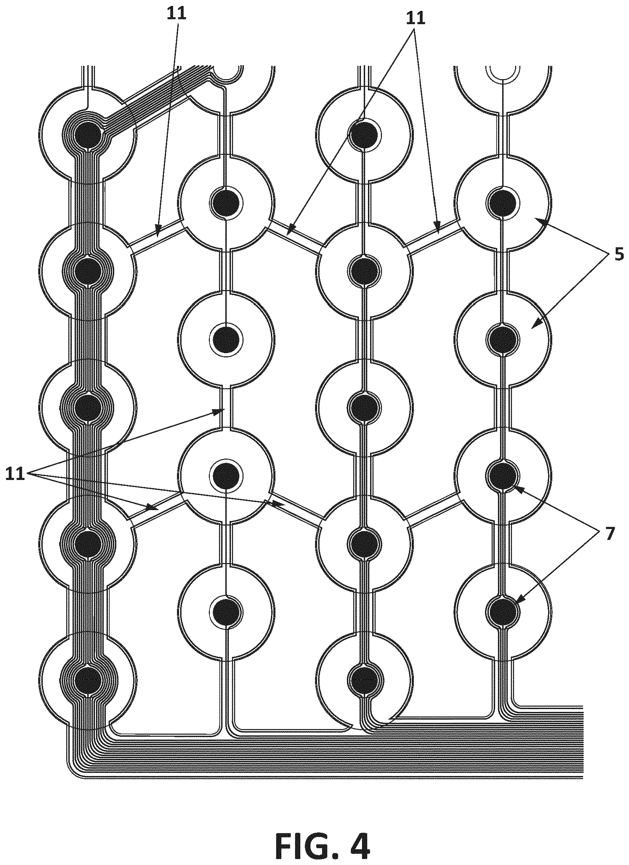

In other words, the direction followed by the different conductive paths is in general not uniform (e.g., from down to up or from left to right). In fact, the conductive paths are preferably routed so that the portions connecting the nodes in the outermost part of the mesh have a larger number of conductive paths than those located in the innermost part. In this way, the innermost nodes contain several portions with a low number of conductive paths, so that they can be removed for example to enable attachment of other medical equipment, such as defibrillators or catheter navigation systems, interrupting a minimal number of conductive paths. Any portion 6 interconnecting nodes 5 can be cut, which will interrupt only those conductive paths routed through the cut portion. As a matter of example, in FIG. 4, a piece of a sensor array 1-4, 1', 2' is shown in detail. Like in FIGs. 1a-f, substrate portions connect pairs of substrate nodes 5. However, some substrate portions 11 do not have electrically conductive paths. Therefore, if they are cut in order to remove or reposition one or more nodes 5, such cuttings do not break any conductive path of any remaining electrodes 5. In other words, portions 11 not having conductive paths may be torn without affecting the operation of any electrode 7.

Electrode arrays 1-4, 1', 2' can be adapted in size in order to match the size of the user or a determined anatomical structure (for example, the torso of the user). With this purpose, portions 6 connecting two substrate nodes 5 can be cut to enable the removal or repositioning of electrodes 7 in a plurality of manners. In general, any single or group of electrodes can be removed from the electrode array by cutting through corresponding portions 6. The sensor array 1-4, 1', 2' is designed in such a way that several electrodes 7 can be removed by cutting certain portions 6 without affecting the functioning of other electrodes 7, because many portions 6 -specially, portions located in the inner area of the electrode array, contain only one or none electrical path. Therefore, when nodes (electrodes) connected to neighboring ones by portions of these characteristics (having one or none electrical path), the operation of the remaining electrodes in the array is minimally affected. Thus, electrode arrays are adaptable in size because substrate portions having none or very few conductive paths can be torn, broken or separated, in such a way that a group of electrodes of the sensor array is separated and discarded. Besides, when groups of nodes (and corresponding electrodes) are removed from the electrode array, the relative position of nodes which were originally connected to currently removed nodes can be adapted with more freedom, thanks to the size and shape of the nodes and to the flexible portions interconnecting nodes. Therefore, the relative location of electrodes 7 can be modified thanks to portions 6 of flexible material interconnecting pairs of nodes 5, or by cutting or tearing some flexible portions connecting pairs of nodes 5 which do not contain electrically conducting paths. And very often this cutting does not affect the electrical connection of electrodes many inner portions 6 carry zero or at most one electrical connection.

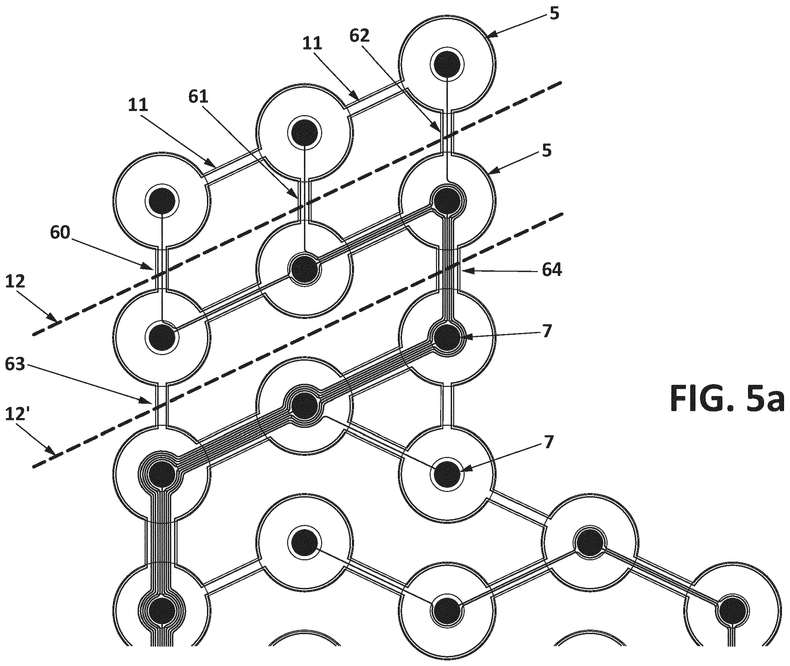

In FIG. 5a, which represents a detailed portion of the device of FIG. 1b, the conductive paths of the uppermost nodes are designed to define two exemplary, possible cutting zones 12, 12'. By cutting the sensor array by a cutting zone, certain sensors can be removed from the array without affecting other sensors in the array. For example, by cutting the sensor array by cutting zone 12, the three portions 60, 61, 62 are cut, and therefore the three distal electrodes are removed from the sensor array. Instead, by cutting the sensor array by cutting zone 12', the two portions 63, 64 are cut, and therefore the six distal electrodes are removed from the sensor array. In both cases, by cutting through zones 12, 12' some conductive paths are cut, but this does not imply that electrodes in the sensor array lose their functionality, since the interrupted conductive paths were paths associated to removed electrodes. This enables to adapt the sensor array 1-4, 1', 2' to patients with shorter sizes, without affecting the functioning of the electrodes 7 which are more proximal to the connector 8, beneath the cutting zone 12, 12'. Therefore, some electrodes 7 can be removed by cutting flexible portions 6 connecting pairs of nodes 5, without affecting the functioning of any other electrode. For example, electrodes located at the distal end of the sensor array with respect to connector 8 can be removed by cutting flexible portions 6 connecting pairs of electrodes, without affecting the functioning of electrodes more proximal to the connector 8. Cutting zones 12, 12' are only exemplary ones. Many other cutting zones can be defined in the electrode arrays of the invention.

When the portions to be cut or teared are portions that do not contain conductive paths at all, they can be cut without interrupting any conductive path. A large variation in the relative positions of the remaining nodes is enabled without electrically isolating any operative electrode. Portions not containing conductive paths help to maintain the structure of the mesh, improving its handling and affixing to a patient.

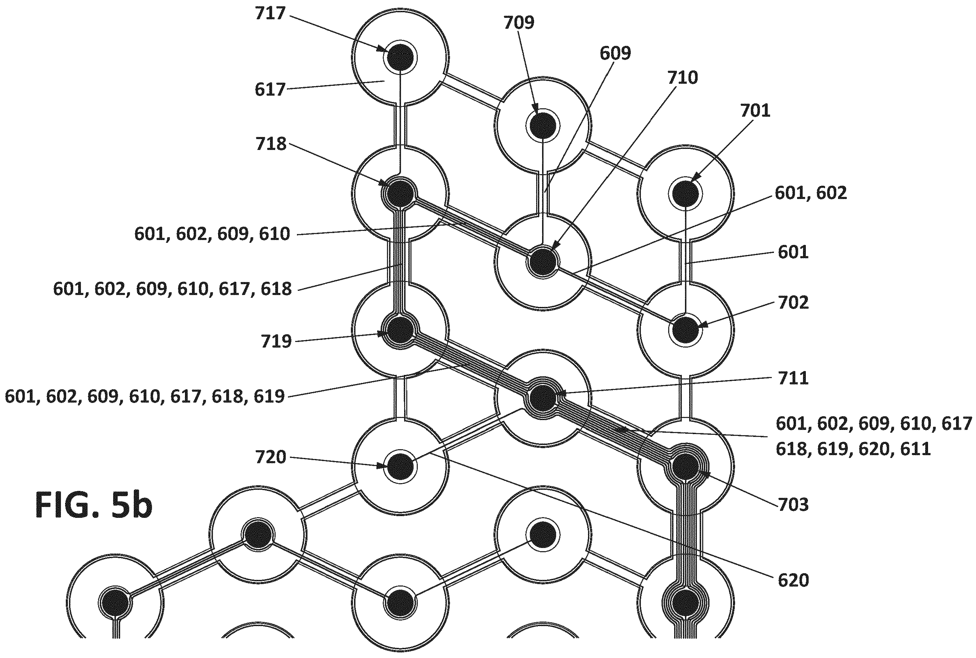

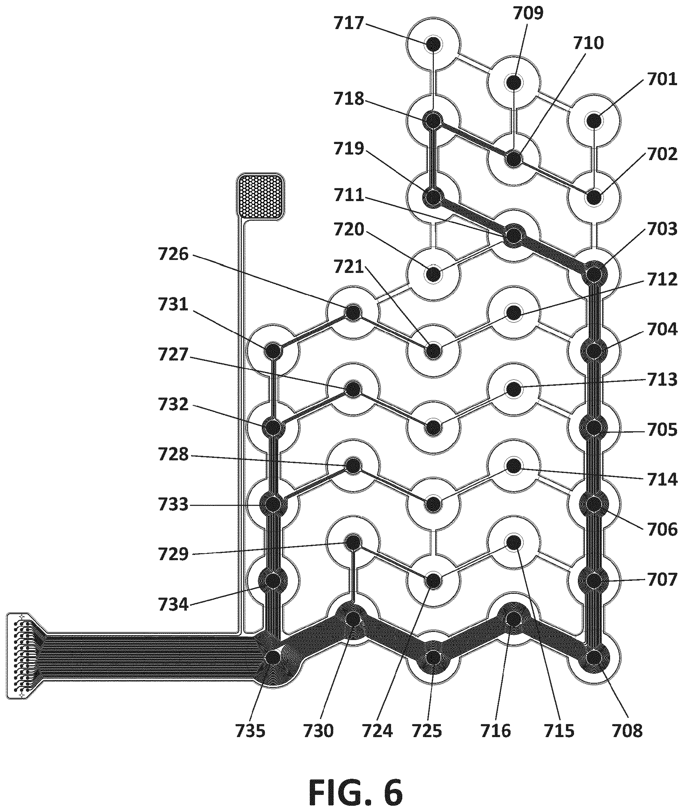

The routing of electrically conductive paths in the particular embodiment of electrode array 1 is explained in detail with reference to FIG. 6. For illustrative purposes, FIG. 5b shows a detailed portion of the array 1 of FIGs. 1a and 6, in which some nodes and corresponding electrodes, portions and conductive paths are shown. The portion interconnecting electrodes 701 and 709 does not have a conductive path. The portion interconnecting electrodes 709 and 717 neither has a conductive path. Neither between electrodes 702 and 703. The portion interconnecting electrodes 701 and 702 has one conductive path 601. The same applies to portion interconnecting electrodes 709 and 710 (conductive path 609); and to portion interconnecting electrodes 717 and 718 (conductive path 617). Between electrodes 702 and 710 there are two conductive paths 601, 602 (for electrically connecting with connector 8 electrodes 701 and 702, respectively). Between electrodes 710 and 718 there are four conductive paths 601, 602, 609, 610 (for electrically connecting with connector 8 electrodes 701, 702, 709 and 710, respectively). Portion 6 between electrodes 710 and 718 is therefore wider than the previous ones. Between electrodes 718 and 719 there are six conductive paths 601, 602, 609, 610, 617, 618 (for electrically connecting with connector 8 electrodes 701, 702, 709, 710, 717 and 718 respectively). Portion 6 between electrodes 718 and 719 is therefore wider than the previous ones. The portion interconnecting electrodes 719 and 720 does not have a conductive path. The portion interconnecting electrodes 720 and 726 neither has a conductive path. Between electrodes 719 and 711 there are seven conductive paths 601, 602, 609, 610, 617, 618, 619 (for electrically connecting with connector 8 electrodes 701, 702, 709, 710, 717, 718 and 719, respectively). Portion 6 between electrodes 719 and 711 is therefore wider than the previous ones. The portion interconnecting electrodes 720 and 711 has one conductive path 620. Between electrodes 711 and 703 there are nine conductive paths 601, 602, 609, 610, 617, 618, 619, 620, 611 (for electrically connecting with connector 8 electrodes 701, 702, 709, 710, 717, 718, 719, 720 and 711, respectively). Portion 6 between electrodes 711 and 703 is therefore wider than the previous ones.

As shown in FIG. 6, the portion interconnecting electrodes 712 and 704 does not have a conductive path. The portion interconnecting electrodes 713 and 705 neither has a conductive path. Neither between electrodes 714 and 706. Nor between electrodes 715 and 707. Therefore, between electrodes 703 and 704 there are ten conductive paths (for electrically connecting with connector 8 electrodes 701, 702, 709, 710, 717, 718, 719, 720, 711 and 703, respectively). Between electrodes 704 and 705 there are eleven conductive paths (for electrically connecting with connector 8 electrodes 701, 702, 709, 710, 717, 718, 719, 720, 711, 703 and 704, respectively). Between electrodes 705 and 706 there are twelve conductive paths (for electrically connecting with connector 8 electrodes 701, 702, 709, 710, 717, 718, 719, 720, 711, 703, 704 and 705, respectively). Between electrodes 706 and 707 there are thirteen conductive paths (for electrically connecting with connector 8 electrodes 701, 702, 709, 710, 717, 718, 719, 720, 711, 703, 704, 705 and 706, respectively). Between electrodes 707 and 708 there are fourteen conductive paths (for electrically connecting with connector 8 electrodes 701, 702, 709, 710, 717, 718, 719, 720, 711, 703, 704, 705, 706 and 707, respectively). Between electrodes 708 and 716 there are fifteen conductive paths (for electrically connecting with connector 8 electrodes 701, 702, 709, 710, 717, 718, 719, 720, 711, 703, 704, 705, 706, 707 and 708, respectively). Between electrodes 716 and 725 there are sixteen conductive paths (for electrically connecting with connector 8 electrodes 701, 702, 709, 710, 717, 718, 719, 720, 711, 703, 704, 705, 706, 707, 708 and 716, respectively). Between electrodes 725 and 730 there are seventeen conductive paths (for electrically connecting with connector 8 electrodes 701, 702, 709, 710, 717, 718, 719, 720, 711, 703, 704, 705, 706, 707, 708, 716 and 725, respectively).

The portion interconnecting electrodes 712 and 721 has one conductive path. Between electrodes 721 and 726 there are two conductive paths (for electrically connecting with connector 8 electrodes 712 and 721, respectively). Between electrodes 726 and 731 there are three conductive paths (for electrically connecting with connector 8 electrodes 712, 721 and 726, respectively). Between electrodes 731 and 732 there are four conductive paths (for electrically connecting with connector 8 electrodes 712, 721, 726 and 731, respectively).

The portion interconnecting electrodes 713 and 722 has one conductive path. Between electrodes 722 and 727 there are two conductive paths (for electrically connecting with connector 8 electrodes 713 and 722, respectively). Between electrodes 727 and 732 there are three conductive paths (for electrically connecting with connector 8 electrodes 713, 722 and 727, respectively). Between electrodes 732 and 733 there are eight conductive paths (for electrically connecting with connector 8 electrodes 712, 721, 726, 731, 713, 722, 727 and 732, respectively).

The portion interconnecting electrodes 714 and 723 has one conductive path. Between electrodes 723 and 728 there are two conductive paths (for electrically connecting with connector 8 electrodes 714 and 723, respectively). Between electrodes 728 and 733 there are three conductive paths (for electrically connecting with connector 8 electrodes 714, 723 and 728, respectively). Between electrodes 733 and 734 there are twelve conductive paths (for electrically connecting with connector 8 electrodes 712, 721, 726, 731, 713, 722, 727, 732, 714, 723, 728 and 733, respectively). Between electrodes 734 and 735 there are thirteen conductive paths (for electrically connecting with connector 8 electrodes 712, 721, 726, 731, 713, 722, 727, 732, 714, 723, 728, 733 and 734, respectively).

The portion interconnecting electrodes 723 and 724 does not have a conductive path. The portion interconnecting electrodes 715 and 724 has one conductive path. Between electrodes 724 and 729 there are two conductive paths (for electrically connecting with connector 8 electrodes 715 and 724, respectively). Between electrodes 729 and 730 there are three conductive paths (for electrically connecting with connector 8 electrodes 715, 724 and 729, respectively). Between electrodes 730 and 735 there are twenty-one electrodes (for electrically connecting with connector 8 electrodes 701, 702, 709, 710, 717, 718, 719, 720, 711, 703, 704, 705, 706, 707, 708, 716, 725, 715, 724, 729 and 730, respectively).

Finally, in portion 10 there are thirty-five electrodes (for electrically connecting with connector 8 electrodes 701, 702, 709, 710, 717, 718, 719, 720, 711, 703, 704, 705, 706, 707, 708, 716, 725, 715, 724, 729, 730, 712, 721, 726, 731, 713, 722, 727, 732, 714, 723, 728, 733, 734, 735 and 9, respectively). Portion 10 can contain an additional conductive path for connecting the reference node (9 with connector 8).

As can be seen, many portions 6 can be cut without losing electrical connectivity with any electrodes. This is the case if portions between electrodes 701 and 709; or between electrodes 709 and 717; or between electrodes 719 and 720; or between electrodes 720 and 726; or between electrodes 704 and 712; or between electrodes 705 and 713; or between electrodes 706 and 714; or between electrodes 707 and 715; or between electrodes 723 and 724; are cut. This enables high repositioning of electrodes without reducing the number of electrodes in the array.

Also, groups of electrodes can be removed from the array without provoking the electrical isolation of electrodes remaining in the array. For example, if portion between electrodes 718 and 719 is cut, electrodes 701, 702, 709, 710, 717 and 718 are removed, but all remaining electrodes maintain their conductive path to connector 8. Similarly, if portions between electrodes 717 and 718, between 709 and 710, and between 701 and 702 are cut, electrodes 701, 709 and 717 are removed, but all remaining electrodes maintain their conductive path to connector 8. Similarly, if portions between electrodes 705 and 713, between 706 and 714, between 707 and 715, between 722 and 727, between 723 and 728, and between 724 and 729 are cut, electrodes 713-715 and 722-724 are removed, but all remaining electrodes maintain their conductive path to connector 8. These are only a few examples of possible reconfiguration and repositioning enabled by the electrode array of the invention.

A similar detailed description of conductive paths and reconfiguration and cutting possibilities can be made, mutatis mutandis, with the electrode arrays 2-4, 1', 2' of FIGs. 1b-f. Such detailed description is not repeated for conciseness, but can be followed from the former description together with the electrically conductive paths illustrated in FIG. 6.

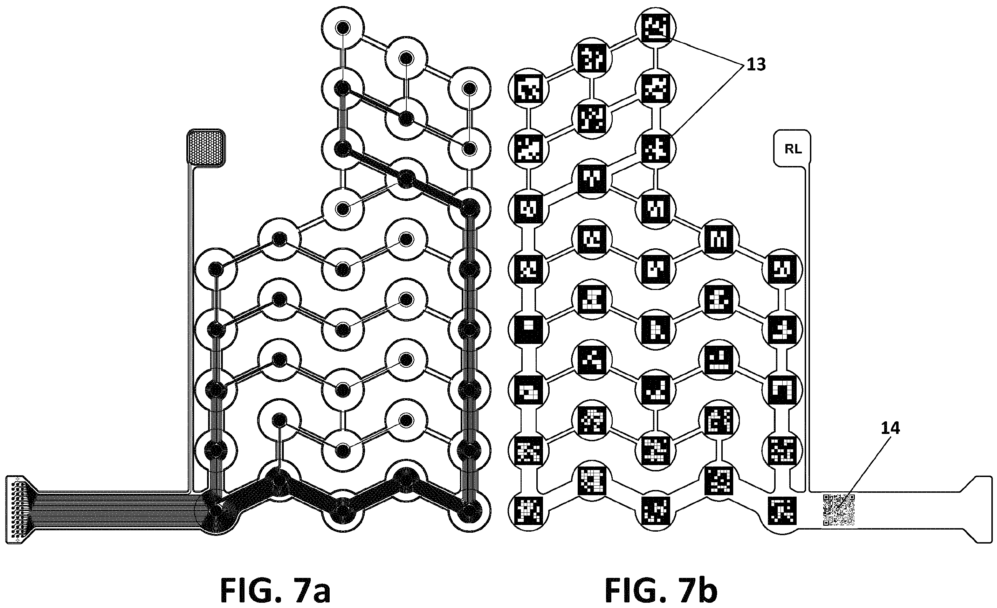

In embodiments of the invention, at least some of the electrodes of each electrode array 1-4, 1', 2' have a unique identifier associated thereto. For example, each electrode 7 may have one unique identifier associated thereto. In embodiments of the invention, the unique identifier associated to an electrode is a visual code 13, such as a QR or an ArUCo. The unique code can be used for the automatic identification of the position and/or orientation of the electrode using image analysis or computer vision techniques. An example of unique identifier associated to individual electrodes (or substrate nodes) is shown in FIG. 7b, in which each node has respective unique identifiers 13 (in this case, QR codes). Because each code is unique, it permits the automatic identification of the position of the electrode to which it is associated. The codes also enable the identification of the orientation of the electrodes, for example by calculating the normal with respect to the plane in which the unique code is located. The unique code associated to an electrode can be printed in or attached (for example adhered) to the substrate node 5 in which each electrode 7 is placed. In embodiments of the invention, the sensor array 1-4, 1', 2' can also have a unique identifier (i.e., code) 14 identifying the array. This identifier may be printed in or attached to the sensor array 1-4, 1', 2' , for example in the vicinity of connector 8, as shown in FIG. 7b. Visual codes enable the automatic identification of a sensor position and/or orientation without the need of medical imaging systems.

A new sensor array has been disclosed, which has large sensor repositioning capabilities, achieved by a combination of features: small individual substrate nodes of flexible material for holding electrodes (one electrode per substrate node); substrate portions of flexible material interconnecting substrate nodes in the mesh, wherein each node is only interconnected to two or three neighbouring nodes; and absence of electrically conductive paths in some of the substrate portions interconnecting neighbouring substrate nodes. This allows for example to modify the density of electrodes on an anatomical structure and to adapt to different patients' complexions. Portions interconnecting nodes can be cut. When these portions do not carry electrical paths, the operation of the whole array of electrodes is maintained in spite of one or more portions having been cut.

The new sensor array also enables to remove several individual electrodes without affecting the operation of other electrodes, thanks to the absence of electrically conductive paths in some of the substrate portions interconnecting neighbouring substrate nodes. This provides even larger repositioning capabilities and allows to use the sensor array together with other systems that require direct access to the anatomical structure under study.

What is more, groups of electrodes can be removed from the sensor array without affecting the operation of other electrodes even when removing electrodes implies cutting some electrically conductive paths. This is achieved thanks to the specific design of the electrical routes. It is possible to remove groups of electrodes 7 without affecting the functioning of other electrodes electrically disposed between the cutting point (cutting area) and the connector. Besides, unique visual codes permit identifying the position and/or orientation of individual electrodes using non-medical image approaches, as well as identifying the sensor array. These codes make the use of other techniques or materials unnecessary, for example specific radio-opaque materials and medical imaging techniques.

## Claims

1. A device (1-4, 1', 2') for measuring electrical potentials of a body part of a patient, comprising:
a plurality of substrate nodes (5) made of a flexible material, the plurality of substrate nodes (5) being configured to be disposed on the torso of the patient, wherein some pairs of substrate nodes of the plurality of substrate nodes (5) are interconnected by straight portions (6) of flexible material, the plurality of substrate nodes (5) and the straight portions (6) of flexible material forming a flexible substrate, wherein there is one straight portion (6) per pair of substrate nodes (5) to be interconnected,
a plurality of electrodes (7), wherein each electrode of the plurality of electrodes (7) is disposed on a respective substrate node (5) of the plurality of substrate nodes (5), and
at least one connector (8),
wherein each electrode of the plurality of electrodes (7) is connected to the at least one connector (8) through a respective conductive path, wherein each conductive path is embedded in the flexible substrate formed by substrate nodes (5) and straight portions (6), and wherein the device (1-4, 1', 2') is
configured to remove one or more substrate nodes (5) and corresponding electrodes (7) by cutting the portions (6) which connect the one or more substrate nodes (5) and corresponding electrodes (7) with the remaining substrate nodes in the device (1-4, 1', 2'), without interrupting the conductive paths of the remaining electrodes in the device (1-4, 1', 2'), **characterized in that**
each conductive path travelling from the electrode (7) to be connected to the at least one connector (8), to said at least one connector (8), is travelling through a plurality of substrate nodes (5) and straight portions (6), and **in that**
some of the straight portions (6) connecting two substrate nodes (5) have an embedded conductive path travelling from one substrate node to the other substrate node (5), and some of the straight portions (6) connecting two substrate nodes (5) do not have an embedded conductive path travelling from one substrate node to the other substrate node (5).

2. The device of claim 1, wherein the substrate nodes (5) have circular shape.

3. The device of any one of claims 1-2, wherein each substrate node (5) is connected to between two and six adjacent nodes (5) by a respective number of straight portions (6).

4. The device of any one of the preceding claims, wherein groups of substrate nodes (5) and respective electrodes (7) are aligned forming columns.

5. The device of claim 4, wherein neighbor substrate nodes (5) and respective electrodes (7) belonging to different columns are disposed in zig-zag.

6. The device of any one of the preceding claims, wherein for all inner substrate nodes (5), each substrate node (5) is surrounded by six neighboring substrate nodes, each neighboring substrate node being disposed in a corner of an imaginary hexagon centered around the substrate node (5).

7. The device of any one of the preceding claims, configured for placement on the anterior portion of a user's torso, the device (1, 2, 1', 2') comprising five columns of substrate nodes (5) and corresponding electrodes (7), wherein a first column has five substrate nodes, a second column has five substrate nodes, a third column has nine substrate nodes, a fourth column has eight substrate nodes and a fifth column has eight substrate nodes.

8. The device of any one of claims 1-6, configured for placement on the posterior portion of a user's torso, the device (3-4) comprising four columns of substrate nodes (5) and corresponding electrodes (7), wherein a first column has five substrate nodes, a second column has eight substrate nodes, a third column has eight substrate nodes and a fourth column has eight substrate nodes.

9. The device of any one of the preceding claims, wherein the width of each portion (6) connecting two substrate nodes (5) depends on the number of conductive paths it carries.

10. The device of any one of the preceding claims, wherein the at least one connector (8) is connected to an acquisition system for further processing of the sensed electrical potentials.

11. The device of any one of the preceding claims, further comprising a substrate portion (10) disposed between the at least one connector (8) and one of the substrate nodes (5), said substrate portion (10) carrying a portion of all the conductive paths connecting the electrodes (7) with the at least one connector (8).

12. The device of any one of the preceding claims, further comprising a unique identifier (13) associated with at least some of the electrodes (7), the unique identifier (13) enabling automatic identification of the location of electrode with which it is associated.

13. The device of claim 12, wherein the unique identifier is a visual code.

14. The device of any one of the preceding claims, further comprising at least one additional substrate node and corresponding electrode (9) serving as electrical reference.

15. A system comprising at least two devices (1-4, 1', 2') according to any one of the preceding claims.

## Patentansprüche

1. Vorrichtung (1-4, 1', 2') zur Messung elektrischer Potentiale eines Körperteils eines Patienten, umfassend:
eine Vielzahl von Substratknoten (5) aus flexiblem Material, wobei die Vielzahl von Substratknoten (5) derart ausgebildet ist, dass sie auf dem Torso des Patienten angeordnet werden kann, wobei einige Paare von Substratknoten der Vielzahl von Substratknoten (5) durch gerade Abschnitte (6) aus flexiblem Material miteinander verbunden sind, wobei die Vielzahl von Substratknoten (5) und die geraden Abschnitte (6) aus flexiblem Material ein flexibles Substrat bilden, wobei für jedes Paar von zu verbindenden Substratknoten (5) ein gerader Abschnitt (6) vorgesehen ist,
eine Vielzahl von Elektroden (7), wobei jede Elektrode der Vielzahl von Elektroden (7) auf einem jeweiligen Substratknoten (5) der Vielzahl von Substratknoten (5) angeordnet ist, und
mindestens einen Anschluss (8),
wobei jede Elektrode der Vielzahl an Elektroden (7) über einen jeweiligen leitfähigen Pfad mit dem mindestens einen Anschluss (8) verbunden ist, wobei jeder leitfähige Pfad in dem durch Substratknoten (5) und gerade Abschnitte (6) gebildeten flexiblen Substrat eingebettet ist, und
wobei die Vorrichtung (1-4, 1', 2') derart ausgebildet ist, dass ein oder mehrere Substratknoten (5) und zugehörige Elektroden (7) entfernt werden können durch Durchtrennen der Abschnitte (6), die den einen oder mehrere Substratknoten (5) und zugehörige Elektroden (6) mit den verbleibenden Substratknoten der Vorrichtung (1-4, 1', 2') verbinden, ohne dass die leitfähigen Pfade der verbleibenden Elektroden der Vorrichtung (1-4, 1', 2') unterbrochen werden, **dadurch gekennzeichnet,**
**dass** jeder leitfähige Pfad, verlaufend von der Elektrode (7) verbindend zu dem mindestens einen Anschluss (8), durch eine Vielzahl von Substratknoten (5) und geraden Abschnitten (6) verläuft, und
**dass** einige der geraden Abschnitte (6), die zwei Substratknoten (5) miteinander verbinden, einen eingebetteten leitfähigen Pfad aufweisen verlaufend von einem Substratknoten zu einem anderen Substratknoten (5), und einige der geraden Abschnitte (6), die zwei Substratknoten (5) miteinander verbinden, keinen eingebetteten leitfähigen Pfad verlaufend von einem Substratknoten zu einem anderen Substratknoten (5) aufweisen.

2. Vorrichtung nach Anspruch 1, wobei die Substratknoten (5) kreisförmig ausgebildet sind.

3. Vorrichtung nach einem der Ansprüche 1 oder 2, wobei jeder Substratknoten (5) mit zwei bis sechs benachbarten Substratknoten (5) durch eine entsprechende Anzahl gerader Abschnitte (6) verbunden ist.

4. Vorrichtung nach einem der vorhergehenden Ansprüche, wobei Gruppen von Substratknoten (5) und zugehörigen Elektroden (7) derart ausgerichtet sind, dass sie Reihen bilden.

5. Vorrichtung nach Anspruch 4, wobei benachbarte Substratknoten (5) und zugehörige Elektroden (7), die zu verschiedenen Reihen gehören, in Zickzack-Anordnung angeordnet sind.

6. Vorrichtung nach einem der vorhergehenden Ansprüche, wobei bei allen inneren Substratknoten (5) jeder Substratknoten (5) von sechs benachbarten Substratknoten umgeben ist, wobei jeder benachbarte Substratknoten in einer Ecke eines gedachten Hexagons angeordnet ist, das um den Substratknoten (5) zentriert ist.

7. Vorrichtung nach einem der vorhergehenden Ansprüche, die zur Anbringung auf dem anterioren Teil des Torsos eines Benutzers ausgebildet ist, wobei die Vorrichtung (1, 2, 1', 2') fünf Reihen von Substratknoten (5) und zugehörigen Elektroden (7) umfasst, wobei eine erste Reihe fünf Substratknoten aufweist, eine zweite Reihe fünf Substratknoten aufweist, eine dritte Reihe neun Substratknoten aufweist, eine vierte Reihe acht Substratknoten aufweist, und eine fünfte Reihe acht Substratknoten aufweist.

8. Vorrichtung nach einem der Ansprüche 1 bis 6, die zur Anbringung auf dem posterioren Teil des Torsos eines Benutzers ausgebildet ist, wobei die Vorrichtung (3-4) vier Reihen von Substratknoten (5) und zugehörigen Elektroden (7) umfasst, wobei eine erste Reihe fünf Substratknoten aufweist, eine zweite Reihe acht Substratknoten aufweist, eine dritte Reihe acht Substratknoten aufweist, und eine vierte Reihe acht Substratknoten aufweist.

9. Vorrichtung nach einem der vorhergehenden Ansprüche, wobei die Breite jedes Abschnitts (6), der zwei Substratknoten (5) verbindet, von der Anzahl der von ihm geführten leitfähigen Pfade abhängt.

10. Vorrichtung nach einem der vorhergehenden Ansprüche, wobei der mindestens eine Anschluss (8) mit einem Aufnahmesystem zur weiteren Verarbeitung der erfassten elektrischen Potentiale verbunden ist.

11. Vorrichtung nach einem der vorhergehenden Ansprüche, weiterhin umfassend einen Substratabschnitt (10), der zwischen dem mindestens einen Anschluss (8) und einem der Substratknoten (5) angeordnet ist, wobei der Substratabschnitt (10) einen Teil aller leitfähigen Pfade führt, die die Elektroden (7) mit dem mindestens einen Anschluss (8) verbinden.

12. Vorrichtung nach einem der vorhergehenden Ansprüche, weiterhin umfassend eine eindeutige Kennung (13), die mindestens einigen der Elektroden (7) zugeordnet ist, wobei die eindeutige Kennung (13) eine automatische Identifizierung der Position der ihr zugeordneten Elektrode ermöglicht.

13. Vorrichtung nach Anspruch 12, wobei die eindeutige Kennung ein visueller Code ist.

14. Vorrichtung nach einem der vorhergehenden Ansprüche, weiterhin umfassend mindestens einen zusätzlichen Substratknoten und eine zugehörige Elektrode (9), die als elektrische Referenz dienen.

15. System, umfassend mindestens zwei Vorrichtungen (1-4, 1', 2') nach einem der vorhergehenden Ansprüche.

## Revendications

1. Dispositif (1-4, 1', 2') pour mesurer des potentiels électriques d'une partie corporelle d'un patient, comprenant :
une pluralité de nœuds de substrat (5) faits d'un matériau flexible, la pluralité de nœuds de substrat (5) étant configurée pour être disposée sur le torse du patient, dans laquelle certaines paires de nœuds de substrat de la pluralité de nœuds de substrat (5) sont reliées entre elles par des parties droites (6) de matériau flexible, la pluralité de nœuds de substrat (5) et les parties droites (6) de matériau flexible formant un substrat flexible, dans lequel il y a une partie droite (6) par paire de nœuds de substrat (5) à relier entre elles,
une pluralité d'électrodes (7), dans laquelle chaque électrode de la pluralité d'électrodes (7) est disposée sur un nœud de substrat (5) respectif de la pluralité de nœuds de substrat (5), et
au moins un connecteur (8),
dans lequel chaque électrode de la pluralité d'électrodes (7) est connectée au au moins un connecteur (8) par le biais d'une piste conductrice respective, dans lequel chaque piste conductrice est intégrée dans le substrat flexible formé par des nœuds de substrat (5) et des parties droites (6), et dans lequel le dispositif (1-4, 1', 2') est conçu pour éliminer un ou plusieurs nœuds de substrat (5) et les électrodes (7) correspondantes en coupant les parties (6) qui relient les un ou plusieurs nœuds de substrat (5) et les électrodes (7) correspondantes avec les nœuds de substrat restants dans le dispositif (1-4, 1', 2') sans interrompre les pistes conductrices des électrodes restantes dans le dispositif (1-4, 1', 2'), **caractérisé en ce que**
chaque piste conductrice allant de l'électrode (7) à connecter au au moins un connecteur (8) jusqu'audit au moins un connecteur (8) passe à travers une pluralité de nœuds de substrat (5) et de parties droites (6), et **en ce que**
certaines des parties droites (6) reliant deux nœuds de substrat (5) ont une piste conductrice intégrée allant d'un nœud de substrat à l'autre nœud de substrat (5) et certaines des parties droites (6) reliant deux nœuds de substrat (5) n'ont pas de piste conductrice intégrée passant d'un nœud de substrat à l'autre nœud de substrat (5).

2. Dispositif selon la revendication 1, dans lequel les nœuds de substrat (5) ont une forme circulaire.

3. Dispositif selon l'une quelconque des revendications 1 à 2, dans lequel chaque nœud de substrat (5) est relié à entre deux et six nœuds (5) adjacents par un nombre respectif de parties droites (6).

4. Dispositif selon l'une quelconque des revendications précédentes, dans lequel des groupes de nœuds (5) et des électrodes (7) respectives sont alignés, formant des colonnes.

5. Dispositif selon la revendication 4, dans lequel les nœuds de substrat (5) voisins et les électrodes (7) respectives appartenant à des colonnes différentes sont disposés en zigzag.

6. Dispositif selon l'une quelconque des revendications précédentes, dans lequel, pour tous les nœuds de substrat internes (5), chaque nœud de substrat (5) est entouré par six nœuds de substrat voisins, chaque nœud de substrat voisin étant disposé dans un coin d'un hexagone imaginaire centré autour du nœud de substrat (5).

7. Dispositif selon l'une quelconque des revendications précédentes, configuré pour être placé sur la partie antérieure du torse d'un utilisateur, le dispositif (1, 2, 1', 2') comprenant cinq colonnes de nœuds de substrat (5) et d'électrodes (7) correspondantes, dans lequel une première colonne a cinq nœuds de substrat, une deuxième colonne a cinq nœuds de substrat, une troisième colonne a neuf nœuds de substrat, une quatrième colonne a huit nœuds de substrat et une cinquième colonne a huit nœuds de substrat.

8. Dispositif selon l'une quelconque des revendications 1 à 6, configuré pour être placé sur la partie postérieure du torse d'un utilisateur, le dispositif (3-4) comprenant quatre colonnes de nœuds de substrat (5) et d'électrodes (7) correspondantes, dans lequel une première colonne a cinq nœuds de substrat, une deuxième colonne a huit nœuds de substrat, une troisième colonne a huit nœuds de substrat et une quatrième colonne a huit nœuds de substrat.

9. Dispositif selon l'une quelconque des revendications précédentes, dans lequel la largeur de chaque partie (6) reliant deux nœuds de substrat (5) dépend du nombre de pistes conductrices qu'elle porte.

10. Dispositif selon l'une quelconque des revendications précédentes, dans lequel le au moins un connecteur (8) est connecté à un système d'acquisition pour le traitement ultérieur des potentiels électriques détectés.

11. Dispositif selon l'une quelconque des revendications précédentes, comprenant en outre une partie de substrat (10) disposée entre le au moins un connecteur (8) et l'un des nœuds de substrat (5), ladite partie de substrat (10) portant une partie de la totalité des pistes conductrices connectant les électrodes (7) avec le au moins un connecteur (8).

12. Dispositif selon l'une quelconque des revendications précédentes, comprenant en outre un identifiant unique (13) associé à au moins certaines des électrodes (7), l'identifiant unique (13) permettant une identification automatique de l'emplacement de l'électrode à laquelle il est associé.

13. Dispositif selon la revendication 12, dans lequel l'identifiant unique est un code visuel.

14. Dispositif selon l'une quelconque des revendications précédentes, comprenant en outre au moins un nœud de substrat additionnel et une électrode (9) correspondante servant de référence électrique.

15. Système comprenant au moins deux dispositifs (1-4, 1', 2') selon l'une quelconque des revendications précédentes.
